# FASCICULE DE BREVET EUROPEEN

(11) **EP 2 341 891 B1**
(45) Date de publication et mention de la délivrance du brevet: **18.06.2014**
(21) Numéro de dépôt: 09749163.3
(22) Date de dépôt: 14.09.2009
(51) Int. Cl.: A61K 8/44, A61K 8/45, A61Q 19/02, C07C 233/45, A61K 31/165, A61P 17/00, C07C 235/34, C07C 237/20

(54) **MONO ESTER DE N-UNDECYLENOYL PHENYLALANINE ET DE POLYOL, PROCEDE POUR SA PREPARATION ET UTILISATION DESDITS ESTERS COMME AGENT ECLAIRCISSANT LA PEAU**
N-UNDECYLENOYL-PHENYLALANIN MONOESTER POLYOL SOWIE DEREN HERSTELLUNG UND VERWENDUNG ALS HAUTBLEICHMITTEL
N-UNDECYLENOYL PHENYLALANINE MONOESTER POLYOL, METHOD OF PREPARATION, AND USE THEREOF AS A SKIN LIGHTENING AGENT

(30) Priorité: 24.09.2008 FR 0856420
(43) Date de publication de la demande: 13.07.2011
(73) Titulaire: Societe D'Exploitation De Produits Pour Les Industries Chimiques Seppic, 75007 Paris (FR)
(72) Inventeur: DUMONT, Sandy, F-81200 Caucalières (FR); TAILLEBOIS, Cécile, F-81150 Castres (FR); GUILBOT, Jérôme, F-81100 Castres (FR); STOLTZ, Corinne, F-94320 Thiais (FR); KERVERDO, Sébastien, F-94300 Vincennes (FR)
(74) Mandataire: Conan, Philippe Claude
(86) Numéro de dépôt international: PCT/FR2009/051717
(87) Numéro de publication internationale: WO 2010/034917

(56) Documents cités:
- EP-A- 0 499 521
- WO-A-03/061768
- US-A1- 2007 282 002

## Description

La présente invention a pour objet un nouvel actif cosmétique apte à éclaircir la peau.

La plupart des formulations cosmétiques éclaircissantes de la peau commercialisées, sont à base d'acide kojique, d'arbutine ou de magnésium ascorbyl phosphate.

La demande de brevet européen publiée sous le numéro EP 1 471 881 divulgue que des dérivés N-acylés d'acides alpha-aminés et notamment le N-undécylénoyl phénylalanine, présentent une affinité vis à vis du récepteur de la Mélanocyte Spécifique Hormone (α-MSH) et induisent ainsi l'éclaircissement de la peau selon le mécanisme biochimique suivant : la compétition entre l'hormone α-MSH et la molécule présentant une affinité vis à vis du récepteur α-MSH, entraîne un moindre taux de fixation de ladite hormone sur les récepteurs cellulaires ; cette compétition a pour conséquence une inhibition de l'activité de l'adénylate cyclase qui entraîne une moindre transformation de l'ATP en AMP cyclique intracellulaire ; la diminution du taux d'AMP cyclique entraîne une inhibition de l'enzyme Protéine Kinase A (PKA) ; l'inhibition de la Protéine Kinase A induit une moindre activation de la tyrosinase du fait de la moindre transformation de cette dernière en tyrosinase phosphorylée ; cette moindre activation de la tyrosinase entraîne la diminution de la synthèse de mélanine d'où découle une moindre pigmentation de la peau.

La demande de brevet japonais N° 2000-229121 décrit l'utilisation des polyols esters des N-acylamino acides comme tensioactifs performants (paragraphe [00003] de ladite demande).

Dans le cadre de leurs recherches de nouveaux actifs éclaircissant la peau, améliorés par rapport à ceux cités ci-dessus, sans détériorer leur tolérance cutanée, les inventeurs ont développé les composés objet de la présente invention.

C'est pourquoi, selon un premier aspect, l'invention a pour objet un composé de formule (I_{b}) : 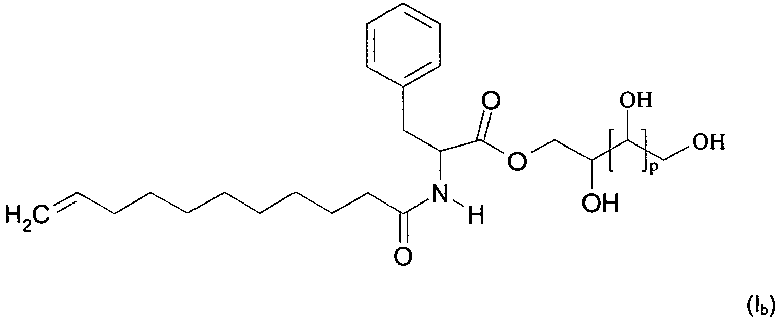 correspondant à la formule (I) telle que définie précédemment, dans laquelle p est un nombre entier égal à 0, 1, 2 ou 3.

Selon un autre aspect particulier de la présente invention, celle-ci a pour objet un composé de formule (I_{b1}) : 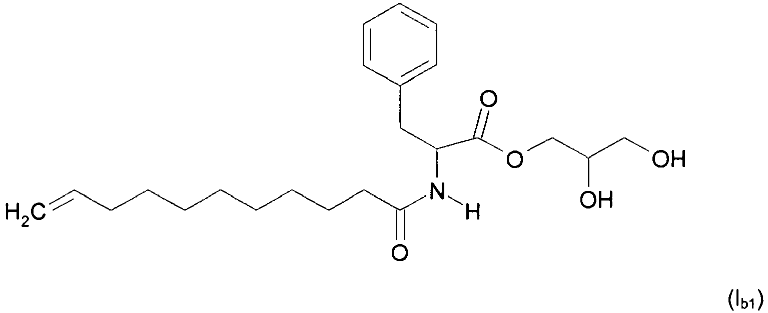 correspondant à la formule (I_{b}) telle que définie précédemment, dans laquelle p est égal à 0.

Selon un autre aspect particulier de la présente invention, celle-ci a pour objet un composé de formule (I_{b2}): 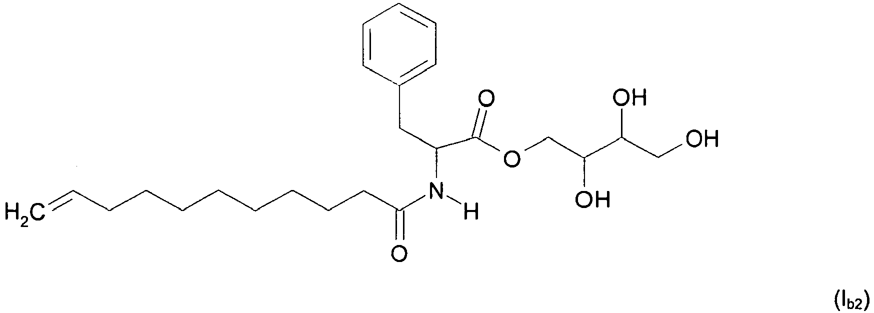 correspondant à la formule (I_{b}) telle que définie précédemment, dans laquelle p est égal à 1.

Selon un autre aspect particulier de la présente invention, celle-ci a pour objet un composé de formule (I_{b3}) : 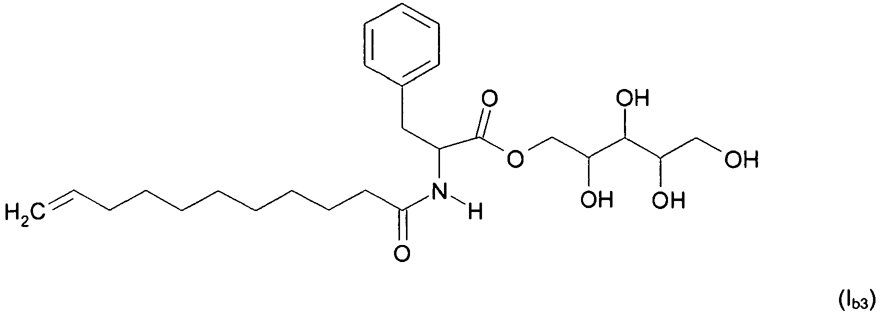 correspondant à la formule (I_{b}) telle que définie précédemment, dans laquelle p est égal à 2.

Selon un autre aspect particulier de la présente invention, celle-ci a pour objet un composé de formule (I_{b4}) : 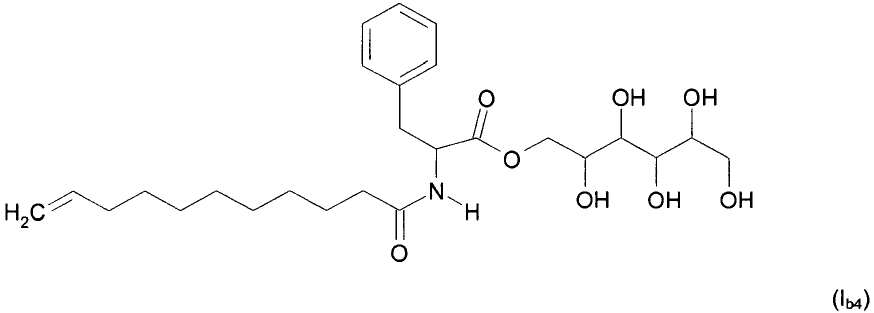 correspondant à la formule (I_{b}) telle que définie précédemment, dans laquelle p est égal à 3.

L'invention a aussi pour objet un procédé de préparation du composé de formule (Ib) telle que définie précédemment comprenant :
une étape a) d'estérification du composé de formule (II) : 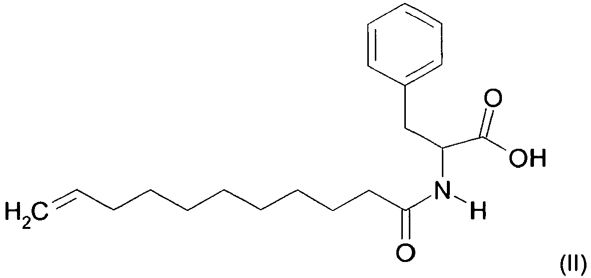 avec le polyol de formule (III): 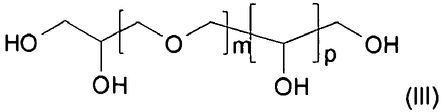 dans laquelle m est égal à 0 et p est un nombre entier égal à 0, 1, 2 ou 3,
   pour former le composé de formule (Ib) et éventuellement un composé de formule (IV) 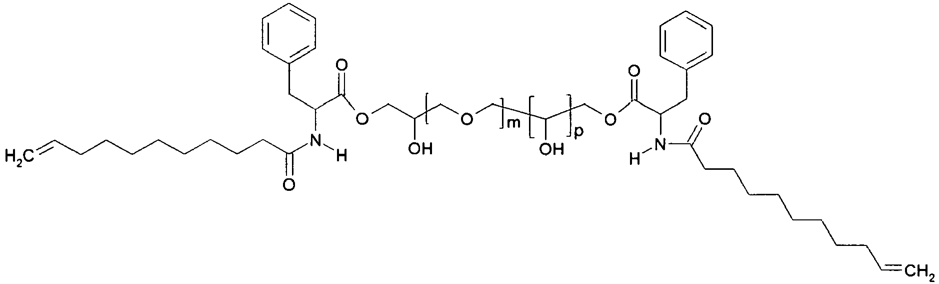 dans laquelle m est égal à 0 et p est un nombre entier égal à 0, 1, 2 ou 3, et si désiré
une étape b) de séparation dudit composé de formule (IV) et dudit composé de formule (I).

Dans le procédé tel que défini ci-dessus, l'étape a) est généralement effectuée à une température d'environ 100°C sous gaz inerte et en catalyse acide.

Lors de cette étape a), il est possible que se forme aussi, mais dans une moindre proportion que le composé de formule (Ib), le composé de formule (IV). Le ratio molaire composé de formule (Ib) sur composé de formule (IV) obtenus lors de l'étape a) du procédé est généralement supérieur ou égal à 80/20 et souvent supérieur ou égal à 90/10.

L'étape b) de séparation des composés de formule(Ib) et de formule (IV) est mise oeuvre par les méthodes classiques de séparation connues de l'homme du métier.

Selon un autre aspect, l'invention a pour objet une composition cosmétique caractérisée en ce qu'elle comprend comme agent éclaircissant de la peau du corps humain, une quantité efficace d'un composé de formule (Ib) telle que définie précédemment.

Selon un autre aspect, l'invention a pour objet un composé de formule (Ib) telle que définie précédemment, pour son utilisation comme actif éclaircissant de la peau du corps humain.

Dans la composition et/ou le médicament tels que définis ci-dessus, le composé de formule (Ib) est généralement mis en oeuvre en une quantité comprise entre 0,05 % massique et 10 % massique pour 100% de la masse de la composition, plus particulièrement entre 0,5 % massique et 5 % massique pour 100% de la masse de la composition et tout particulièrement entre 1% massique et 5% massique pour 100% de la masse de la composition.

Les compositions et/ou médicaments tels que définis ci-dessus, se présentent généralement sous forme de solutions aqueuses ou hydro-alcooliques diluées, sous forme d'émulsions simples ou multiples, telles que les émulsions eau dans huile (E/H), huile dans eau (H/E), huile dans eau dans huile (H/E/H) ou eau dans huile dans eau (E/H/E), dans lesquelles l'huile est de nature végétale et/ou animale et/ou minérale, ou sous forme de poudre. Elles peuvent aussi être dispersées ou imprégnées sur du textile ou sur des matériaux non tissés, qu'il s'agisse de lingettes, de serviettes en papier ou de vêtements.

Les compositions et/ou médicaments tels que définis ci-dessus, sont administrées au sujet sous les formes classiques utilisées en cosmétique et en pharmacie ; il s'agit plus particulièrement de l'administration par voie topique.

De façon générale, un composé de formule (Ib) telle que définie précédemment, seul ou en mélange avec un composé de formule (IV) telle que définie précédemment, est associé à de nombreux types d'adjuvants ou de principes actifs utilisés dans les formulations cosmétiques, qu'il s'agisse, de corps gras, de solvants organiques, d'épaississants, de gélifiants, d'adoucissants, d'antioxydants, d'opacifiants, de stabilisants, de tensioactifs moussants et/ou détergents, d'émollients, de parfums, d'émulsionnants ioniques ou non, de charges, de séquestrants, de chélateurs, de conservateurs, d'huiles essentielles, de matières colorantes, de pigments, d'actifs hydrophiles ou lipophiles, d'humectants, comme par exemple la glycérine, de conservateurs, de colorants, d'actifs cosmétiques, de filtres solaires minéraux et/ou organiques, de charges minérales comme les oxydes de fer, oxydes de titane et le talc, de charges synthétiques comme par exemple les nylons et les poly(méthacrylate de méthyle) réticulés ou non, d'élastomères silicone, de séricites ou d'extraits de plantes ou encore de vésicules lipidiques ou tout autre ingrédient habituellement utilisé en cosmétique.

Comme exemples d'huiles que l'on peut associer à un composé de formule (Ib) telle que définie précédemment, seul ou en mélange avec un composé de formule (IV) telle que définie précédemment, on peut citer les huiles minérales telles que l'huile de paraffine, l'huile de vaseline, les isoparaffines ou les huiles blanches minérales ; les huiles d'origine animale, telles que le squalène ou le squalane ; les huiles végétales, telles que l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula ; les huiles végétales éthoxylées ; les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les polyalphaoléfines, les polyoléfines comme le polyisobutène, les isoalcanes de synthèse comme l'isohexadecane , identifié dans Chemical Abstracts par le numéro RN = 93685-80-4 et qui est un mélange d'isoparaffines en C₁₂, C₁₆ et C₂₀ contenant au moins 97% d'isoparaffines en C₁₆, parmi lesquelles le constituant principal est le 2,2,4,4,6,8,8-heptaméthyl nonane (RN = 4390-04-9), l'isododécane, le polydécène hydrogéné ou le polyisobutène hydrogéné, commercialisé en France par la société Ets B. Rossow et Cie sous le nom PARLEAM - POLYSYNLANE™, cité dans : Michel and Irene Ash ; Thesaurus of Chemical Products, Chemical Publishing Co, Inc. 1986 Volume I, page 211 (ISBN 0 7131 3603 0), les huiles perfluorées ; et les huiles de silicone comme les diméthylpolysiloxanes, méthylphénylpolysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.

Comme autre matière grasse que l'on peut associer à un composé de formule (Ib) telle que définie précédemment, seul ou en mélange avec un composé de formule (IV) telle que définie précédemment, on peut citer les alcools gras saturés ou insaturés, linéaires ou ramifiés, ou les acides gras saturés ou insaturés, linéaires ou ramifiés.

Parmi les polymères épaississants et/ou émulsionnants utilisés dans la présente invention, il y a par exemple les polymères de type polyélectrolytes comme par exemple les homopolymères ou copolymères de l'acide acrylique ou de dérivés de l'acide acrylique, les homopolymères ou copolymères de l'acide méthacrylique ou de dérivés de l'acide méthacrylique, les homopolymères ou copolymères de l'acrylamide, les homopolymères ou copolymères de dérivés de l'acrylamide, les homopolymères ou copolymères de l'acide 2-méthyl [(1-oxo 2-propényl) amino] 1-propanesulfonique (AMPS), les homopolymères ou copolymères de monomères vinyliques, les homopolymères ou copolymères de chlorure de triméthylaminoéthylacrylate ; les hydrocolloïdes d'origine végétale ou biosynthétique, par exemple la gomme de xanthane, la gomme de karaya, les carraghénates, les alginates, les galactomannanes ; les silicates ; la cellulose et ses dérivés ; l'amidon et ses dérivés hydrophiles ; les polyuréthanes.

Parmi les polymères de type polyélectrolytes, pouvant être mis en jeu dans la production d'une phase aqueuse gélifiée apte à être utilisée dans la préparation d'émulsions E/H, H/E, E/H/E ou H/E/H, ou d'un gel aqueux contenant le composé de formule (I) telle que définie précédemment, seul ou en mélange avec un composé de formule (IV) telle que définie précédemment, il y a par exemple, les copolymères de l'acide acrylique et de l'acide 2-méthyl [(1-oxo 2-propényl) amino] 1-propanesulfonique (AMPS), les copolymères de l'acrylamide et de l'acide 2-méthyl [(1-oxo 2-propényl) amino] 1-propanesulfonique, les copolymères de l'acide 2-méthyl [(1-oxo 2-propényl) amino] 1-propanesulfonique et de l'acrylate de (2-hydroxyéthyle), l'homopolymère de l'acide 2-méthyl [(1-oxo 2-propényl) amino] 1-propanesulfonique, l'homopolymère de l'acide acrylique, les copolymères de l'acide acrylique et du N-[2-hydroxy-1,1-bis(hydroxyméthyl) éthyl] propénamide [ou tris(hydroxyméthyl) acrylamidométhane ou N-tris(hydroxyméthyl) méthyl acrylamide dénommé aussi THAM], les copolymères de l'AMPS et du THAM, les copolymères du chlorure d'acryloyl éthyl triméthyl ammonium et de l'acrylamide, les copolymères de l'AMPS et de la vinylpyrolidone, les copolymères de l'acide acrylique et d'acrylates d'alkyle dont la chaîne carbonée comprend entre dix et trente atomes de carbone, les copolymères de l'AMPS et d'acrylates d'alkyle dont la chaîne carbonée comprend entre dix et trente atomes de carbone. De tels polymères sont commercialisés respectivement sous les appellations SIMULGEL™ EG, SEPIGEL™ 305, SIMULGEL™ NS, SIMULGEL™ 800, SIMULGEL™ A, SIMULGEL™ EPG, SIMULGEL™ INS 100, SIMULGEL™ FL, SIMULGEL™ SMS 88, SEPISOFT™ SP, SEPIGEL™ 501, SEPIGEL™ 502, SEPIPLUS™ 250, SEPIPLUS™ 265, SEPIPLUS™ 400, SEPINOV™ EMT 10, CARBOPOL™, ULTREZ™ 10, ACULYN™, PEMULEN™ TR1, PEMULEN™ TR2, LUVIGEL™ EM, SALCARE™ SC91, SALCARE™ SC92, SALCARE™ SC95, SALCARE™ SC96, FLOCARE™ ET100, FLOCARE™ ET58, HISPAGEL™, NOVEMER™ EC1, ARISTOFLEX™ AVC, ARISTOFLEX™ HBM, RAPITHIX™ A60, RAPITHIX™ A100, COSMEDIA SP, STABILEZE™ 06 et STABILEZE™ QM.

Parmi les cires utilisables dans la présente invention, on peut citer par exemple la cire d'abeille ; la cire de carnauba ; la cire de candelilla ; la cire d'ouricoury ; la cire du Japon ; la cire de fibre de liège ou de canne à sucre ; les cires de paraffines ; les cires de lignite ; les cires microcristallines ; la cire de lanoline ; l'ozokérite ; la cire de polyéthylène ; les huiles hydrogénées ; les cires de silicone ; les cires végétales ; les alcools gras et les acides gras solides à température ambiante ; les glycérides solides à température ambiante.

Parmi les émulsionnants utilisables dans la présente invention, on peut citer les esters gras d'alkylpolyglycosides éventuellement alcoxylés, et tout particulièrement les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120 ; les esters gras alcoxylés tels que le PEG 150 pentaérythrytyl tétrastéarate commercialisé sous l'appellation CROTHIX™ DS53, le PEG 55 propylène glycol oléate commercialisé sous l'appellation ANTIL™ 141 ; les carbamates de polyalkylène glycols à chaînes grasses tels que le PPG 14 laureth isophoryl dicarbamate commercialisé sous l'appellation ELFACOS™ T211, le PPG 14 palmeth 60 hexyl dicarbamate commercialisé sous l'appellation ELFACOS™ GT2125 ; les acides gras, les acides gras éthoxylés, les esters d'acide gras et de sorbitol, les esters d'acides gras éthoxylés, les polysorbates, les esters de polyglycérol, les alcools gras éthoxylés, les esters de sucrose, les alkylpolyglycosides, les alcools gras sulfatés et phosphatés ou les mélanges d'alkylpolyglycosides et d'alcools gras décrits dans les demandes de brevet français 2 668 080, 2 734 496, 2 756 195, 2 762 317, 2 784 680, 2 784 904, 2 791 565, 2 790 977, 2 807 435, 2 804 432, 2 830 774, 2 830 445, les associations de tensioactifs émulsionnants choisis parmi les alkylpolyglycosides, les associations d'alkylpolyglycosides et d'alcools gras, les esters de polyglycérols ou de polyglycols ou de polyols tels que les polyhydroxystéarates de polyglycols ou de polyglycérols mis en oeuvre dans les demandes de brevets français 2 852 257, 2 858 554, 2 820 316 et 2 852 258.

Parmi les tensioactifs moussants et/ou détergents utilisables dans la présente invention, on peut citer : les tensioactifs anioniques, cationiques, amphotères ou non ioniques topiquement acceptables habituellement utilisés dans ce domaine d'activité.

Parmi les tensioactifs anioniques utilisables dans la présente invention, on citera particulièrement les sels de métaux alcalins, les sels de métaux alcalino-terreux, les sels d'ammonium, les sels d'amines, les sels d'aminoalcools des composés suivants : les alkyléthers sulfates, les alkylsulfates, les alkylamidoéthersulfates, les alkylarylpolyéthersulfates, les monoglycérides sulfates, les alpha-oléfinesulfonates, les paraffines sulfonates, les alkylphosphates, les alkylétherphosphates, les alkylsulfonates, les alkylamidesulfonates, les alkylarylsulfonates, les alkylcarboxylates, les alkylsulfosuccinates, les alkyléthersulfosuccinates, les alkylamidesulfosuccinates, les alkylsulfoacétates, les alkylsarcosinates, les acyliséthionates, les N-acyltaurates, les acyllactylates. Parmi les tensioactifs anioniques, on citera également les lipoaminoacides, les lipoprotéines, les lipopeptides, les dérivés des lipoprotéines, les dérivés de protéines, les sels d'acides gras, les sels d'acides d'huile de coprah éventuellement hydrogénée.

Parmi les tensioactifs amphotères utilisables dans la présente invention, on citera particulièrement les alkylbétaines, les alkylamidobétaines, les sultaines, les alkylamidoalkylsulfobétaines, les dérivés d'imidazolines, les phosphobétaïnes, les amphopolyacétates et les amphopropionates.

Parmi les tensioactifs cationiques utilisables dans la présente invention, on citera particulièrement les dérivés d'ammoniums quaternaires.

Parmi les tensioactifs non ioniques utilisables dans la présente invention, on citera particulièrement les alkylpolyglycosides, les dérivés d'huile de ricin, les polysorbates, les amides de coprah, les N-alkylamines, les oxydes d'amines.

Comme exemples de principe actif que l'on peut associer au composé de formule (Ib) telle que définie précédemment, seul ou en mélange avec un composé de formule (IV) telle que définie précédemment, on peut citer les autre composés ayant une action éclaircissante ou dépigmentante tels que par exemple l'arbutine, l'acide kojique, l'hydroquinone, l'acide ellagique, la vitamine C et ses dérivés, le magnésium ascorbyl phosphate, le SEPIWHITE™ MSH, le SEPICALM™ VG les extraits de polyphénols, les extraits de raisin, les extraits de pin, les extraits de vin, les extraits d'olives, les extraits de marc, les extraits de jus de pomme, les protéines N-acylées, les peptides N-acylés, les acides aminés N-acylés, comme par exemple le N-lauroyl proline, le N-linoléyl lysine, le N-linoléyl leucine, le N-octanoyl glycine, le N-undécylénoyl phénylalanine, le N-palmitoyl proline, les hydrolysâts partiels de protéines N-acylés, les acides aminés, les peptides, les hydrolysâts totaux de protéines, les hydrolysâts partiels de protéines, les polyols (par exemple, la glycérine ou le butylène glycol), l'urée, l'acide pyrrolidonecarboxylique ou les dérivés de cet acide, l'acide glycyrrhétinique, l'alpha-bisabolol, les sucres ou les dérivés des sucres, les polysaccharides ou leurs dérivés, les hydroxyacides par exemple l'acide lactique, les vitamines, les dérivés de vitamines comme le Rétinol, les dérivés du Rétinol, la vitamine E et ses dérivés, les minéraux, les enzymes, les co-enzymes, comme, le Coenzyme Q10 et ses dérivés, les hormones ou "hormone like", les extraits de soja par exemple, la Raffermine™, les extraits de blé par exemple la Tensine™ ou la Gliadine™, les extraits végétaux, tels que les extraits riches en tanins, les extraits riches en isoflavones ou les extraits riches en terpènes, les extraits d'algues d'eau douce ou marines, les cires essentielles, les extraits bactériens, les minéraux, les lipides en général, les lipides tels que les céramides ou les phospholipides, les actifs ayant une action amincissante comme la caféine ou ses dérivés, les actifs ayant une activité antimicrobienne ou une action purifiante vis à vis des peaux grasses tels que le DEEPALINE™ PVB, le LIPACIDE™ UG, les actifs ayant une propriété énergisante ou stimulante comme par exemple le SEPITONIC™ M3 ou le Physiogényl™, le panthénol et ses dérivés comme le SEPICAP™ MP, les actifs anti-age comme le SEPILIFT™ DPHP, la DEEPALINE™ PVB, le SEPIVINOL™, le SEPIVITAL™, les actifs hydratants comme le SEPICALM™ S, le SEPICALM™ VG et le SEPILIFT™ DPHP, l'AQUAXYL™, le PROTEOL™ SAV 50, les actifs anti-âge, les actifs présentant une action de tenseur ou de lissage immédiat sur la peau comme le SESAFLASH™, les actifs "anti-photo vieillissement", les actifs protecteurs de l'intégrité de la jonction dermo-épidermique, les actifs augmentant la synthèse des composants de la matrice extracellulaire, les actifs ayant une activité amincissante, raffermissante ou drainante comme la caféine, la théophylline, l'Adénosyl Mono Phosphate cyclique (AMPc), le thé vert, la sauge, le ginko biloba, le lierre, le marron d'inde, le bambou, le ruscus, le petit houx, la Centella Asiatica, la bruyère, l'ulmaine, le fucus, le romarin, la saule, les extraits de panais, des actifs créant une sensation de « chauffe » sur la peau comme les activateurs de la microcirculation cutanée (comme par exemple des nicotinates) ou des produits créant une sensation de « fraîcheur » sur la peau (comme par exemple le menthol et ses dérivés), les actifs présentant une action vis à vis des cellules souches, les actifs présentant une action sur l'épiderme, le derme, l'hypoderme et les annexes cutanés (poils, glandes sébacées, pores, ...), les actifs présentant une action vis à vis de la flore cutanée.

Comme filtre solaire que l'on peut incorporer dans la composition selon l'invention, on peut citer tous ceux figurant dans la directive cosmétique 76/768/CEE modifiée annexe VII.

L'invention a aussi pour objet l'utilisation d'un composé de formule (Ib) telle que définie précédemment, comme actif éclaircissant de la peau du corps humain.

L'invention a enfin pour objet un procédé non thérapeutique d'éclaircissement de la peau humaine comprenant au moins une étape d'application sur ladite peau humaine d'une composition cosmétique à usage topique comprenant une quantité efficace d'un composé de formule (Ib), telle que définie précédemment.

Dans le procédé tel que décrit ci-dessus, la composition est étalée sur la surface de la peau à traiter, puis la peau est massée quelques instants.

Comme le montrent les exemples suivants, les composés mis en oeuvre dans les traitements cosmétiques ou thérapeutiques définis précédemment, se caractérisent de façon inattendue, par une activité éclaircissante de la peau dont on peut moduler l'intensité contrairement à celle des compositions de l'état de la technique, comprenant le composé non estérifié par un polyol. Ils sont donc de façon générale appropriés aux traitements destinés à éclaircir la peau notamment par dépigmentation et plus particulièrement pour faire disparaître ou pour atténuer les taches colorées apparaissant sur les peaux âgées.

L'étude expérimentale suivante illustre l'invention sans toutefois la limiter.

### Exemple 1 Préparation du monoglycéride de N-undécylénoyl Phénylalanine [composé A de formule (I_{b1})].

On introduit 780g de N-undécylènoyl phénylalanine, soit un équivalent molaire, dans un réacteur en verre à double enveloppe, dans laquelle circule un fluide caloporteur, muni d'une agitation efficace et d'un dispositif de barbotage d'azote par le fond du réacteur, à une température de 125°C pour permettre la fonte totale du N-undécylènoyl phénylalanine. On introduit par la suite 218g de glycérol, soit un équivalent molaire, sur le N-undécylènoyl phénylalanine à une température d'environ 120°C. Le mélange ainsi préparé est ensuite agité à une vitesse d'agitation d'environ 300 tours/minutes, avec un barbotage d'azote par le fond du réacteur, de façon à obtenir un aspect homogène. Le mélange homogène est maintenu à une température de 120°C pendant 30 minutes, puis 2,2g d'acide hypophosphoreux à 50% et 2,2g d'acide sulfurique à 98% sont introduits dans le mélange homogène préalablement préparée. Le milieu réactionnel est placé sous un vide partiel de 20 mbars (0,2 Pa), et maintenu à une température de 120°C pendant une durée de 20 heures avec évacuation de l'eau formée au moyen d'un montage de distillation. Le milieu réactionnel est ensuite vidangé et les caractéristiques analytiques mesurées sont les suivantes :
Aspect à 20°C : liquide trouble
Indice d'acide (selon NFT 60-204) = 15,3mg KOH/g
Indice d'Hydroxyle (selon U.S. Pharmacopeia XXI NF XVI 01/011985) = 278,3mg KOH/g
Indice de saponification (selon NFT 60-206) = 147,2 mg KOH/g
Indice d'iode (selon NFT 60-203) = 64g I₂/100g
Teneur résiduelle en glycérol (par chromatographie d'exclusion stérique) = 5,6 % Taux de conversion du N-undécylénoyl phényl alanine : ≥ 90 %
Ratio massique monoglycéride obtenu sur diglycéride obtenu : ≥ 85/15
Ratio molaire monoglycéride (PM = 405)/diglycéride (PM = 718) obtenus : ≥ 91/9.

### Exemple 3 : Préparation du monoester de N-undécylénoyl phénylalanine avec l'érythritol [composé C de formule (I_{b}2)].

On opère de manière similaire que l'exemple 1 à partir de 780g de N-undécylènoyl phénylalanine, et de 288g de d'érythritol, soit un équivalent molaire, et on obtient l'ester attendu qui présente les caractéristiques analytiques suivantes :
Aspect à 20°C : Pâte-Solide
Indice d'acide (selon NFT 60-204) = 18,7 mg KOH/g
Indice d'Hydroxyle (selon U.S. Pharmacopeia XXI NF XVI 01/01/1985) = 348,7mg KOH/g
Indice de saponification (selon NFT 60-206) = 139,2 mg KOH/g
Indice d'iode (selon NFT 60-203) = 61 g I₂/100 g

### Exemple 4 : Préparation Préparation du monoester de N-undécylénoyl phénylalanine avec le xylitol [composé D de formule (I_{b3})].

On opère de manière similaire que l'exemple 1 à partir de 780g de N-undécylènoyl phénylalanine, et de 358g de xylitol, soit un équivalent molaire, et on obtient l'ester attendu qui présente les caractéristiques analytiques suivantes :
Aspect à 20°C : Pâte-Solide
Indice d'acide (selon NFT 60-204) = 20,8mg KOH/g
Indice d'Hydroxyle (selon U.S. Pharmacopeia XXI NF XVI 01/01/1985) = 466,9mg KOH/g
Indice de saponification (selon NFT 60-206) = 126,4mg KOH/g
Indice d'iode (selon NFT 60-203) = 52g I₂/100 g

### Exemple 5 : Préparation du monoglycéride de N-undécylénoyl phénylalanine avec le sorbitol [composé E de formule (I_{b4})].

On opère de manière similaire que l'exemple 1 à partir de 780g de N-undécylènoyl phénylalanine, et de 429g de sorbitol, soit un équivalent molaire, et on obtient l'ester attendu qui présente les caractéristiques analytiques suivantes :
Aspect à 20°C : Pâte-Solide
Indice d'acide (selon NFT 60-204) = 21,4mg KOH/g
Indice d'Hydroxyle (selon U.S. Pharmacopeia XXI NF XVI 01/01/1985) = 520,3mg KOH/g
Indice de saponification (selon NFT 60-206) = 120,9mg KOH/g
Indice d'iode (selon NFT 60-203) = 48g I₂/100 g

### Etude de l'activité dépigmentante du composé de formule (Ib) dans des cultures de mélanocytes B16/F1

On a comparé l'influence du monoglycéride de N-undécylénoyl phénylalanine à celle de l'arbutine et du N-undécylénoyl phénylalanine, sur la production de mélanine extracellulaire, dans des cultures de mélanocytes B16/F1.

Les mélanocytes murins de la lignée B16/F1 cultivés en monocouches, sont ensemencés dans des plaques de culture de 96 puits à la densité de 1500 cellules/puits. Les cellules sont cultivées dans un milieu de culture (milieu MCM) à 37°C, dans une atmosphère humide contenant 5 % de CO₂. Les cellules sont utilisées à 50% de confluence. Le milieu MCM a la composition suivante : Milieu DMEM (Dulbecco's Modified Eagle's Medium) contenant 4,5 g/l de glucose additionné de L-Glutamine (2 mM), de pénicilline (50 UI / ml), de streptomycine (50 µg / ml) et de sérum de veau foetal (10% v/v). Le monoglycéride de N-undécylénoyl phénylalanine, l'arbutine et le N-undécylénoyl phénylalanine sont testés à 40 µg / ml dans le milieu MCM. Les cultures de mélanocytes sont incubées en présence du produit à l'essai ou des produits de référence pendant 72 heures à 37°C, dans une atmosphère humide contenant 5 % de CO₂. Des cultures témoins sont incubées, en absence de produit, dans le milieu MCM. Ces cultures témoins sont réalisées sur chaque plaque de culture. Chaque essai est réalisé six fois. Après 72 heures d'incubation, les milieux d'incubation des cellules (n = 6) sont prélevés et stockés à - 80°C jusqu'au moment de l'évaluation des effets. La mélanine extracellulaire est quantifiée par spectrophotométrie à 450 nm. Une gamme d'étalonnage de mélanine est réalisée en parallèle. Les résultats sont exprimés en µg / ml de mélanine extracellulaire et en pourcentage d'inhibition par rapport au groupe témoin dans le Tableau 1 ci-dessous.

### Résultats :

**Tableau 1 : évaluation de l'inhibition de la production de la mélanine extracellulaire des composés selon l'invention et des composés de l'état de la technique.**

| Produits testés | Mélanine extracellulaire (témoin: 98 µg ± 4 µg / ml) | Inhibition de la production de mélanine extracellulaire en % par rapport au témoin |
|---|---|---|
| Arbutine (40 µg / ml) (Etat de la technique) | 39±3 | 61 |
| N-undécylénoyl phénylalanine (PM = 331) (40 µg / ml) (Etat de la technique) | 59±3 | 40 |
| N-undécylénoyl phénylalanine (80 µg / ml) (Etat de la technique) | Cytoxicité | Cytoxicité |
| Composé (A) (40 µg / ml) | 84±8 | 15 |
| Composé (A) (80 µg / ml) | 42 ± 1 | 58 |

Les résultats indiqués dans le Tableau 1 mettent en évidence l'effet dépigmentant induit par le composé A de l'exemple 1. Ils mettent aussi en évidence qu'à concentration molaire en radical N-undécylénoyl phénylalanine équivalente, l'estérification du N-undécylènoyl alanine par le glycérol permet de contrôler l'intensité de l'éclaircissement de la peau contrairement au produit non estérifié

### Etude de l'effet cytotoxique du composé de formule (Ib) dans des cultures de mélanocytes B16/F1

L'évaluation de la présence d'effets cytotoxiques est réalisée en mettant en oeuvre la technique de Bradford par coloration au bleu de Coomassie (Bradford M., «A rapid and sensitive method for the quantification of microgram quantities of protein utilizing the principle of protein of protein-dye binding », Anal. Biochem., 1976, 72, 248-254). Les mélanocytes B16/F1 sont incubés après ensemencement avec les dilutions des produits à l'essai. Après 72 heures d'incubation, les mélanocytes B16/F1 sont lavées puis lysées, et le réactif de Bradford est alors ajouté. Après 5 minutes d'incubation à température ambiante, une lecture spectrophotométrique est réalisée à 640 nm en parallèle d'une gamme d'étalonnage d'albumine. La cytotoxicité est avérée pour une diminution supérieure ou égale à 20 % de la quantité totale de protéine. Les résultats sont exprimés en pourcentage de diminution de la quantité totale de protéines dans le Tableau 2 ci-dessus.

### Résultats :

**Tableau 2: évaluation de la quantité totale de protéines par rapport au témoin (albumine) des composés selon l'invention et des composés de l'état de la technique.**

| Produits testés | Diminution de la quantité totale de protéines en % par rapport au témoin | Evaluation |
|---|---|---|
| N-undécylénoyl phénylalanine (50 µg / ml) (Etat de la technique) | 32% | Cytotoxique |
| N-undécylénoyl phénylalanine (80 µg / ml) (Etat de la technique) | 80% | Cytotoxique |
| Composé A (40 µg / ml) | Pas de diminution observée | Non cytotoxique |
| Composé A (80 µg / ml) | Pas de diminution observée | Non cytotoxique |

Les résultats du Tableau 2 mettent en évidence l'absence d'effets cytotoxiques pour le composé A de l'exemple 1, alors que pour une dose plus faible, le N-undécylénoyl phénylalanine de l'état de la technique se révèle cytotoxique.

Le composé A de l'exemple 1 est donc un composé dépigmentant et non cytotoxique.

## Revendications

1. Composé de formule (I_{b}) : 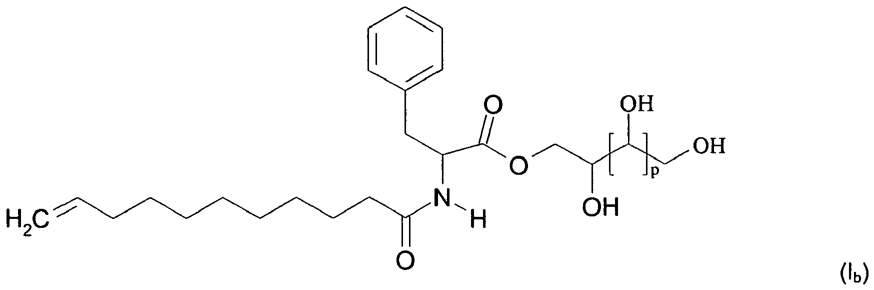 dans laquelle p est un nombre entier égal à 0, 1, 2 ou 3.

2. Composé de formule (I_{b1}) : 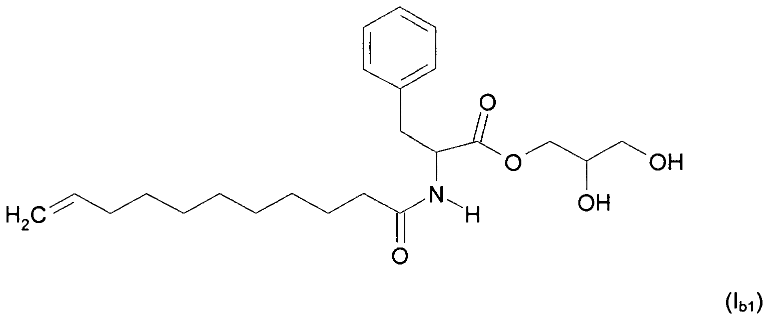 correspondant à la formule (I_{b}) telle que définie à la revendication 1, dans laquelle p est égal à 0.

3. Composé de formule (I_{b2}) : 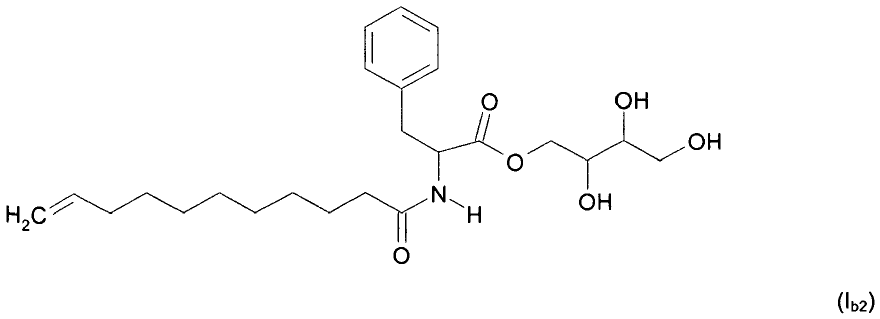 correspondant à la formule (I_{b}) telle que définie à la revendication 1, dans laquelle p est égal à 1.

4. Composé de formule (I_{b3}) : 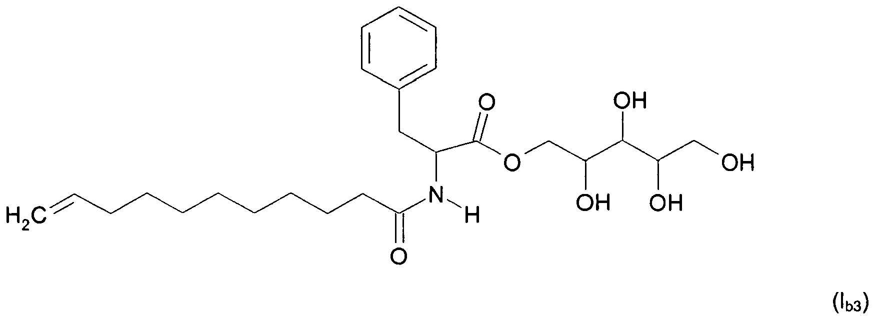 correspondant à la formule (I_{b}) telle que définie à la revendication 1, dans laquelle p est égal à 2.

5. Composé de formule (I_{b4}) : 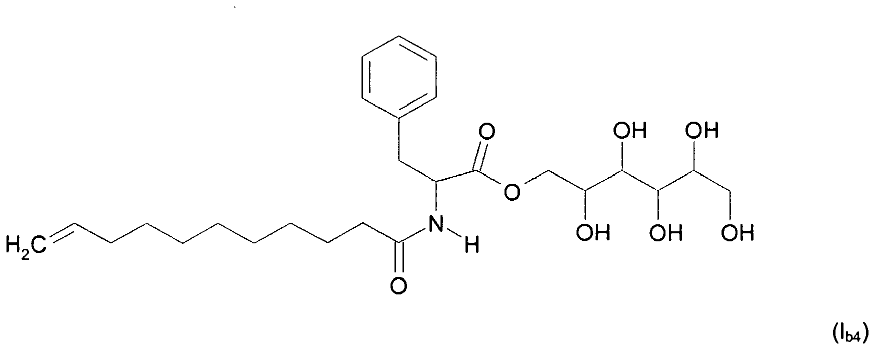 correspondant à la formule (I_{b}) telle que définie à la revendication 1, dans laquelle p est égal à 3.

6. Procédé de préparation du composé de formule (Ib) telle que définie à l'une quelconque des revendications 1 à 5, comprenant :
une étape a) d'estérification du composé de formule (II) : 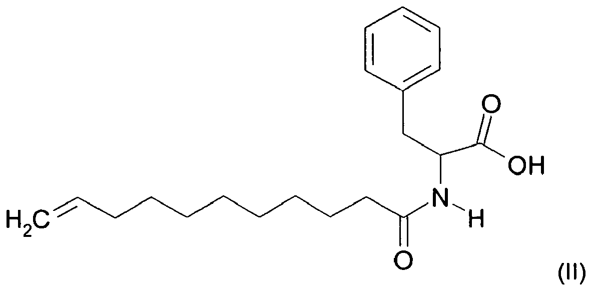 avec le polyol de formule (III) : 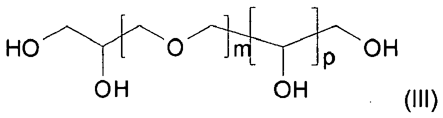 dans laquelle m est un nombre entier égal à 0 et p est un nombre entier égal à 0, 1, 2 ou 3, pour former le composé de formule (Ib) et éventuellement un composé de formule (IV) : 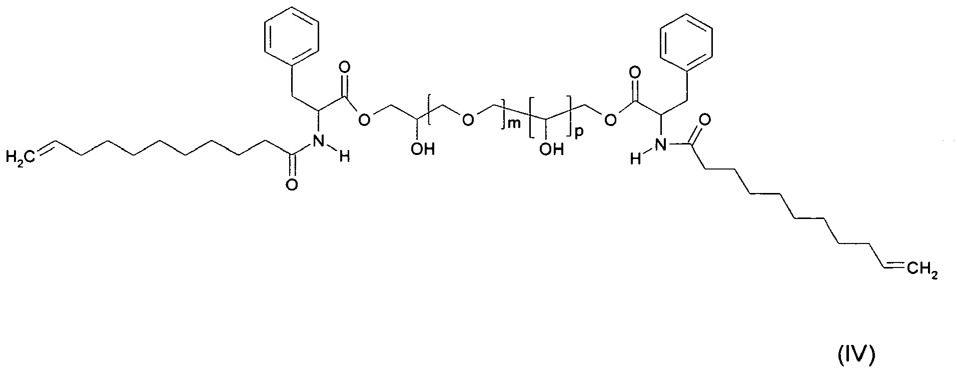 dans laquelle sont tels que définis ci-dessus, et si désiré
une étape b) de séparation dudit composé de formule (IV) et dudit composé de formule (Ib).

7. Composition cosmétique **caractérisée en ce qu'**elle comprend comme agent éclaircissant de la peau du corps humain, une quantité efficace d'un composé de formule (Ib) telle que définie à l'une des revendications 1 à 5.

8. Composé de formule (Ib) telle que définie à l'une de revendiations 1 à 5 pour son utilisation comme médicament à activité éclaircissante de la peau du corps humain.

9. Procédé non thérapeutique d'éclaircissement de la peau humaine comprenant au moins une étape d'application sur ladite peau humaine d'une composition cosmétique à usage topique comprenant une quantité efficace d'un composé de formule (Ib), telle que définie à l'une des revendication 1 à 5.

## Patentansprüche

1. Verbindung der Formel (I_{b}): 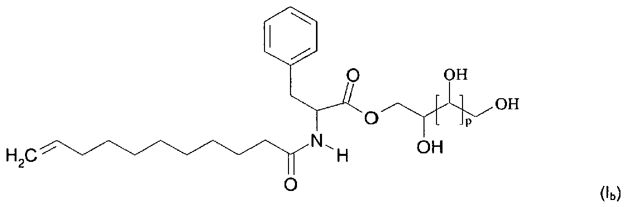 wobei p eine ganze Zahl von 0, 1, 2 oder 3 ist.

2. Verbindung der Formel (I_{b1}): 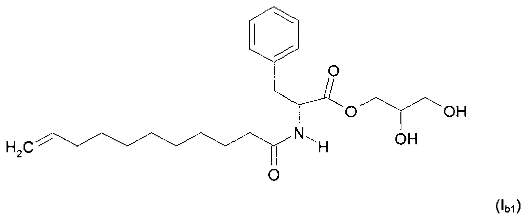 entsprechend Formel (I_{b}) wie in Anspruch 1 definiert, wobei p gleich 0 ist.

3. Verbindung der Formel (I_{b2}): 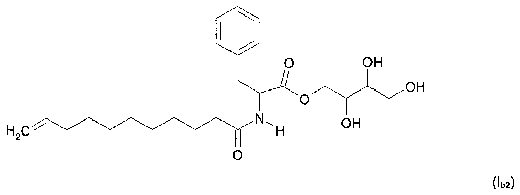 entsprechend Formel (I_{b}) wie in Anspruch 1 definiert, wobei p gleich 1 ist.

4. Verbindung der Formel (I_{b3}): 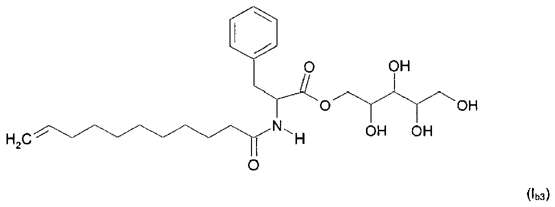 entsprechend Formel (I_{b}) wie in Anspruch 1 definiert, wobei p gleich 2 ist.

5. Verbindung der Formel (I_{b4}): 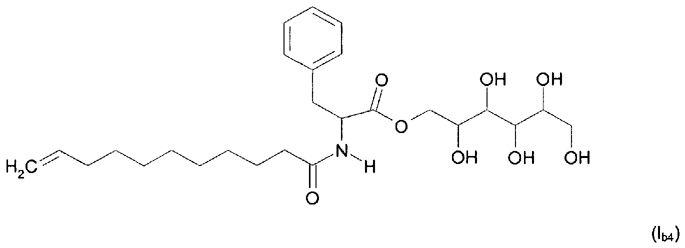 entsprechend Formel (I_{b}) wie in Anspruch 1 definiert, wobei p gleich 3 ist.

6. Verfahren zur Herstellung der Verbindung der Formel (Ib) wie in einem der Ansprüche 1 bis 5 definiert, das Folgendes beinhaltet:
einen Schritt a) des Veresterns der Verbindung der Formel (II): 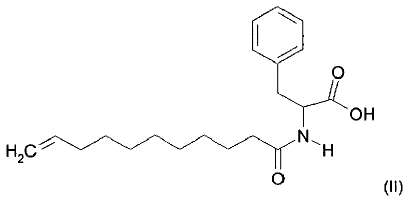 mit dem Polyol der Formel (III): 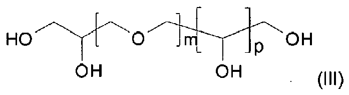 wobei m eine ganze Zahl von gleich 0 ist und p eine ganze Zahl von gleich 0, 1, 2 oder 3 ist, zum Herstellen der Verbindung von Formel (Ib) und eventuell einer Verbindung der Formel (IV): 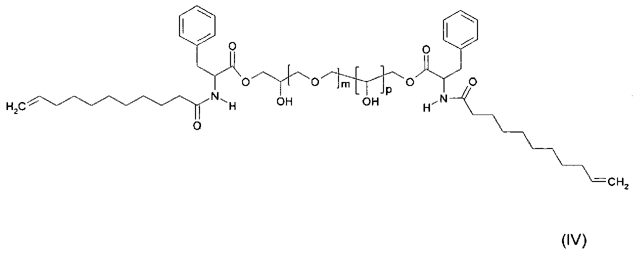 wobei wie oben definiert, und bei Bedarf
einen Schritt b) des Trennens der Verbindung der Formel (IV) und der Verbindung der Formel (Ib).

7. Kosmetikverbindung, **dadurch gekennzeichnet, dass** sie als Mittel zum Aufhellen der Haut des menschlichen Körpers eine wirksame Menge einer Verbindung der Formel (Ib) wie in einem der Ansprüche 1 bis 5 definiert umfasst.

8. Verbindung von Formel (Ib) wie in einem der Ansprüche 1 bis 5 definiert zur Verwendung als Medikament mit aufhellender Wirkung für die Haut des menschlichen Körpers.

9. Nichttherapeutisches Verfahren zum Aufhellen der menschlichen Haut, das wenigstens einen Schritt des Auftragens einer Kosmetikzusammensetzung für den topischen Gebrauch auf die menschliche Haut beinhaltet, die eine wirksame Menge einer Verbindung der Formel (Ib) wie in einem der Ansprüche 1 bis 5 definiert umfasst.

## Claims

1. Compound of formula (I_{b}): 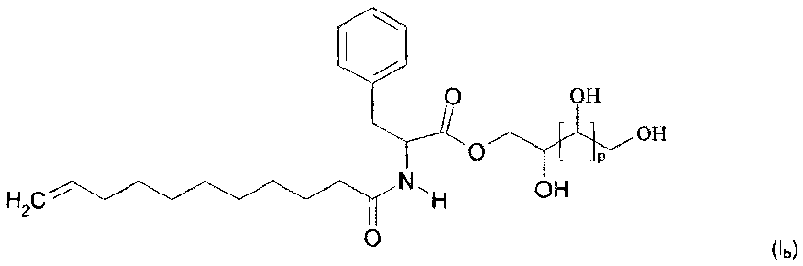 wherein p is an integer equal to 0, 1, 2 or 3.

2. Compound of formula (I_{b1}): 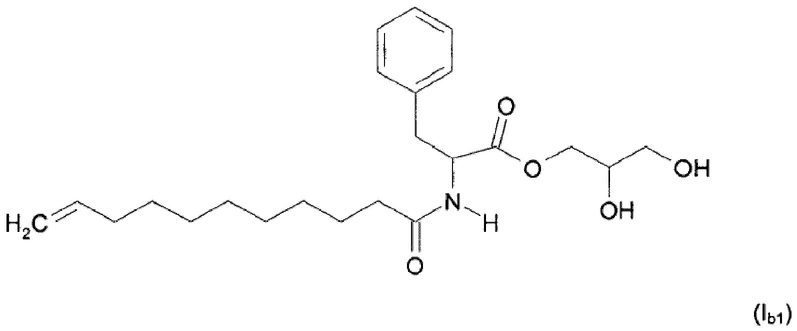 corresponding to formula (I_{b}) according to claim 1, wherein p is equal to 0.

3. Compound of formula (I_{b2}): 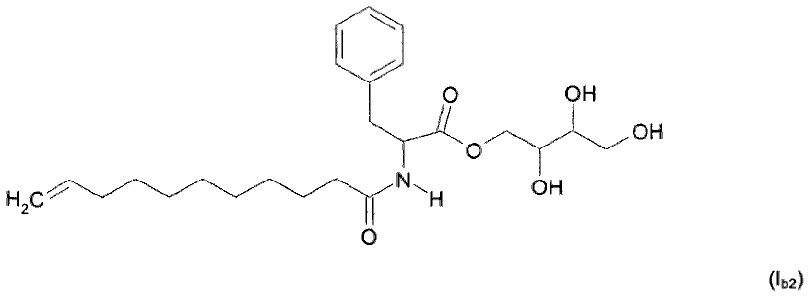 corresponding to formula (I_{b}) according to claim 1, wherein p is equal to 1.

4. Compound of formula (I_{b3}): 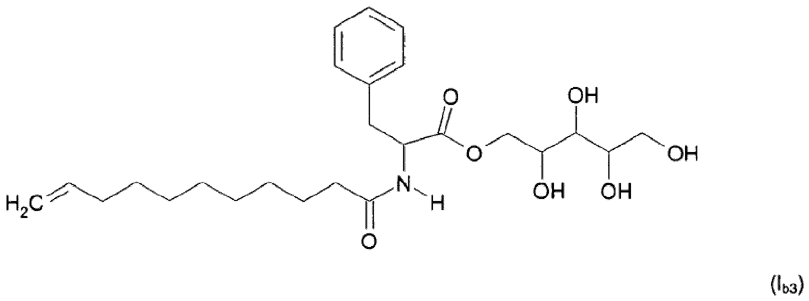 corresponding to formula (I_{b}) according to claim 1, wherein p is equal to 2.

5. Compound of formula (I_{b4}): 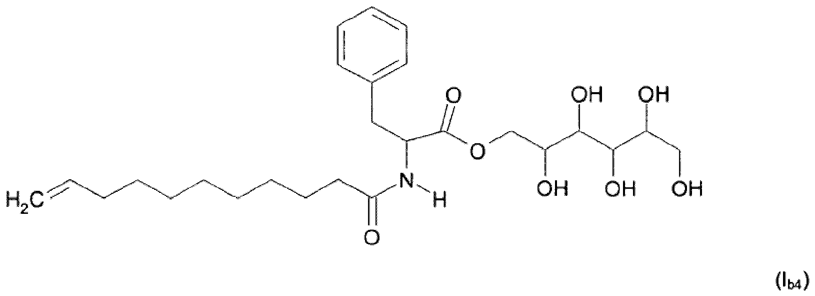 corresponding to formula (I_{b}) according to claim 1, wherein p is equal to 3.

6. Process for preparing the compound of formula (Ib) according to any of claims 1 to 5, comprising:
a step a) of esterifying the compound of formula (II): 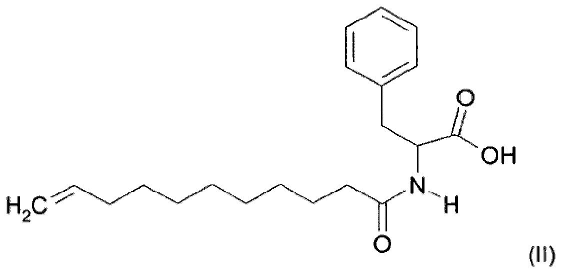 with the polyol of formula (III): 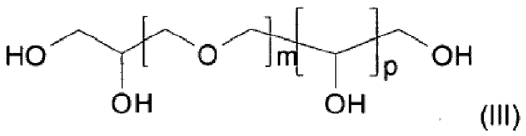 wherein m is an integer equal to 0 and p is an integer equal to 0, 1, 2 or 3, to form the compound of formula (Ib) and optionally a compound of formula (IV): 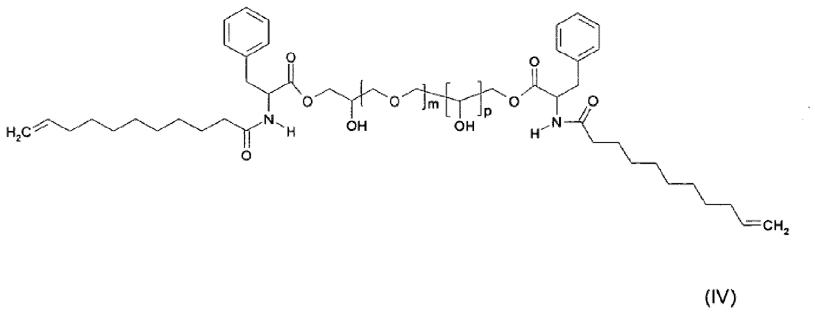 wherein are as defined above, and, if desired,
a step b) of separating said compound of formula (IV) and said compound of formula (Ib).

7. Cosmetic composition, **characterised in that** it comprises as a lightening agent for human skin an effective amount of a compound of formula (Ib) according to any of claims 1 to 5.

8. Compound of formula (Ib) according to any of claims 1 to 5 for use as a medication having lightening activity on human skin.

9. Non-therapeutic process for lightening human skin, comprising at least one step of applying to said human skin a cosmetic composition for topical use comprising an effective amount of a compound of formula (Ib) according to any of claims 1 to 5.
